# FASCICULE DE BREVET EUROPEEN

(11) **EP 0 445 004 B1**
(45) Date de publication et mention de la délivrance du brevet: **05.10.1994**
(21) Numéro de dépôt: 91400474.2
(22) Date de dépôt: 21.02.1991
(51) Int. Cl.: C07D 207/32, A01N 53/00

(54) **Dérivés du pyrrole, leur procédé de préparation et leur application comme pesticides**
Pyrrol-Derivate, Verfahren zu ihrer Herstellung und ihre Anwendung als Pestizide
Pyrrole derivatives, process for their preparation and their use as pesticides

(30) Priorité: 27.02.1990 FR 9002405
(43) Date de publication de la demande: 04.09.1991
(73) Titulaire: ROUSSEL-UCLAF, 75007 Paris (FR)
(72) Inventeur: Benoit, Marc, F-13360 Roquevaire (FR); Demassey, Jacques, F-77144 Montevrain (FR); Demoute, Jean-Pierre, F-93360 Neuilly Plaisance (FR); Tessier, Jean, F-94300 Vincennes (FR)
(74) Mandataire: Tonnellier, Marie-José

(56) Documents cités:
- EP-A- 0 133 406
- EP-A- 0 176 387
- EP-A- 0 335 801
- DE-A- 2 827 627
- FR-A- 2 612 184

## Description

La présente invention concerne de nouveaux dérivés du pyrrole, leur procédé de préparation et leur application comme pesticides.

On connaissait des esters pyréthrinoïdes du 1-(2-propynyl) 1H-pyrrol 3-méthanol doués d'activité insecticide (Cf EP-A-O 176387 et FRA 2612184).

L'invention a pour objet, sous toutes leurs formes stéréoisomères possibles, ainsi que leurs mélanges, les composés de formule (I) : 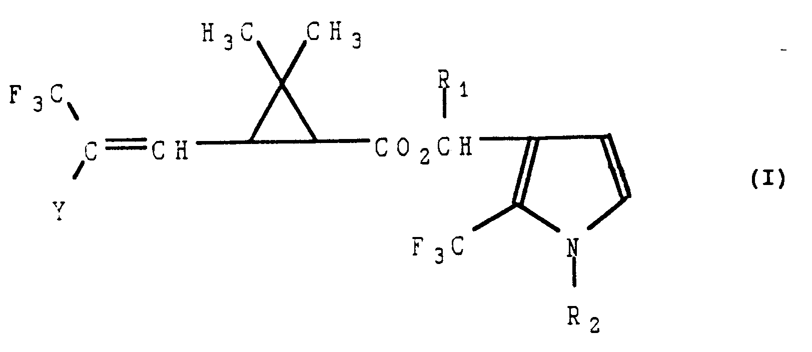
dans lesquels :
- Y représente un atome d'hydrogène, un atome d'halogène, un radical cyano, un radical alkyle éventuellement substitué par un ou plusieurs atomes d'halogène, renfermant jusqu'à 8 atomes de carbone, un radical phényle éventuellement substitué par un ou plusieurs atomes d'halogène et/ou un ou plusieurs radicaux alkyle renfermant jusqu'à 8 atomes de carbone et/ou plusieurs radicaux alkoxy renfermant jusqu'à 8 atomes de carbone,
- R₁ représente un atome d'hydrogène, un radical méthyle, un radical cyano ou un radical éthynyle,
- le radical CF₃ est en position 2, 4 ou 5 sur le noyau pyrrole,
- R₂ représente un radical : 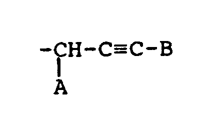 dans lequel A et B identiques ou différents l'un de l'autre, représentent un atome d'hydrogène, un atome d'halogène, un radical alkyle, renfermant jusqu'à 8 atomes de carbone ou un radical aryle renfermant jusqu'à 14 atomes de carbone.

Lorsque Y représente un atome d'halogène, il s'agit de préférence d'un atome de fluor, de chlore ou de brome.

Lorsque Y représente un radical alkyle, il s'agit de préférence d'un radical méthyle, éthyle ou n-propyle.

Lorsque Y représente un radical alkyle substitué par un ou plusieurs atomes d'halogène, il s'agit de préférence d'un radical CF₃ ou CHF₂.

Lorsque Y représente un radical phényle substitué par un ou plusieurs atomes d'halogène, il s'agit de préférence d'un radical 4-chloro phényle.

Lorsque Y représente un radical phényle substitué par un ou plusieurs radicaux alkyle, il s'agit de préférence d'un radical phényle substitué par un ou plusieurs radicaux méthyle, éthyle, n-propyle ou isopropyle.

Lorsque Y représente un radical phényle substitué par un ou plusieurs radicaux alkoxy, il s'agit de préférence d'un radical phényle substitué par un ou plusieurs radicaux méthoxy ou éthoxy.

Lorsque A ou B représente un atome d'halogène, il s'agit de préférence d'un atome de fluor, de chlore ou de brome.

Lorsque A ou B représente un radical alkyle, il s'agit de préférence d'un radical méthyle, éthyle, n-propyle ou isopropyle.

Lorsque A ou B représente un radical aryle, il s'agit de préférence du radical phényle.

L'invention a plus particulièrement pour objet :
- les composés dans lesquels Y représente un atome de chlore,
- les composés dans lesquels R₁ représente un atome d'hydrogène,
- les composés dans lesquels R₂ représente un radical propargyle.

Les composés de formule (I) peuvent exister sous leurs diverses formes stéréoisomères, ainsi que leurs mélanges.

Parmi les composés préférés, on peut citer tout particulièrement les composés dans lesquels la copule cyclopropanique est de structure (1R,cis).

L'invention a tout spécialement pour objet les produits dont la préparation est donnée ci-après dans la partie expérimentale et notamment le composé dont le nom suit :
- [1R-[1alpha,3alpha(Z)]]-3-(2-chloro 3,3,3-trifluoro 1-propényl) 2,2-diméthyl cyclopropanecarboxylate de [1-(2-propynyl) 2-(trifluorométhyl) 1H-pyrrol-3-yl] méthyle,

Les composés de formule (I) présentent d'intéressantes propriétés qui permettent leur utilisation dans la lutte contre les parasites. Il peut s'agir par exemple de la lutte contre les parasites des végétaux, qu'il s'agisse des parasites du sol ou des parties aériennes, les parasites des locaux et les parasites des animaux à sang chaud.

C'est ainsi que l'on peut utiliser les produits de l'invention pour lutter contre les insectes, les nématodes et les acariens parasites des végétaux et des animaux.

L'invention a notamment pour objet l'application des composés de formule (I) à la lutte contre les parasites des végétaux, les parasites des locaux et les parasites des animaux à sang chaud.

Les produits de formule (I) peuvent aussi être utilisés pour lutter contre les insectes et autres parasites du sol, par exemple les coléoptères comme DIABROTICA, les taupins et les vers blancs, les myriapodes comme les scutigérelles et les blaniules, les diptères comme les cécydomies et lès lépidoptères comme les noctuelles terricoles.

Ils sont utilisés à des doses comprises entre 10 g et 300 g de matière active à l'hectare.

Les produits de formule (I) peuvent également être utilisés pour lutter contre les insectes dans les locaux, pour lutter notamment contre les mouches, les moustiques et les blattes.

Les produits de formule (I) sont de plus photostables et sont peu toxiques pour les mammifères.

L'ensemble de ces propriétés fait des produits de formule (I) des produits qui correspondent parfaitement aux exigences de l'industrie agrochimique moderne : ils permettent de protéger les récoltes tout en préservant l'environnement.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques du genre de Boophilus, ceux du genre Hyalomnia, ceux du genre Amblyomnia et ceux du genre Rhipicephalus ou pour lutter contre toutes sortes de gales et notamment la gale sarcoptique, la gale psoroptique et la gale chorioptique.

L'invention a donc également pour objet les compositions destinées à la lutte contre les parasites des animaux à sang chaud, les parasites des locaux et des végétaux, caractérisées en ce qu'elles renferment au moins l'un des produits de formule (I) définis ci-dessus et notamment le produit de l'exemple 1.

L'invention a notamment pour objet les compositions insecticides renfermant comme principe actif au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

Les compositions insecticides selon l'invention contiennent de préférence de 0,005 % à 10 % en poids de matière active.

Selon un mode opératoire avantageux, pour un usage dans les locaux, les compositions selon l'invention sont utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin), amidon et poudre de coque de noix de coco.

La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet les compositions acaricides et nématicides renfermant comme principe actif, au moins un des produits de formule (I), définie ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % de principe actif ou des liquides pour pulvérisation foliaire contenant de 1 à 500 g/l de principe actif. On peut également employer des poudres pour poudrages foliaires contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2.2.1]hept-5-ène-2,3-dicarboximide, ou le pipéronyl-bis-2-(2'-n-butoxy éthoxy) éthylacétal (ou tropital).

Les composés de formule (I) présentent une excellente tolérance générale, et l'invention a donc également pour objet les produits de formule (I), pour lutter notamment contre les affections créées par les tiques et les gales chez l'homme et l'animal.

Les produits de l'invention sont notamment utilisés pour lutter contre les poux à titre préventif ou curatif et pour lutter contre la gale.

Les produits de l'invention peuvent être administrés par voie externe, par vaporisation, par shampooing, par bain ou badigeonnage.

Les produits de l'invention à usage vétérinaire peuvent être également administrés par badigeonnage de l'épine dorsale selon la méthode dite méthode "pour-on".

On peut indiquer également que les produits de l'invention peuvent être utilisés comme biocides ou comme régulateurs de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimidométhylique, d'alcool 5-benzyl 3-furyl méthylique, d'alcools 3-phénoxy benzylique et d'alcools alpha-cyano 3-phénoxy benzyliques des acides chrysanthémiques, par les esters d'alcools 5-benzyl 3-furyl méthylique des acides 2,2-diméthyl 3-(2-oxo 3-tétrahydrothiophénylidène méthyl) cyclopropane-1-carboxyliques, par les esters d'alcool 3-phénoxy benzylique et d'alcools alpha-cyano 3-phénoxy benzyliques des acides 2,2-diméthyl 3-(2,2-dichlorovinyl) cyclopropane-1-carboxyliques, par les esters d'alcools alpha-cyano 3-phénoxy benzyliques d'acides 2,2-diméthyl 3-(2,2-dibromovinyl) cyclopropane-1-carboxyliques, par les esters d'alcools 3-phénoxy benzylique des acides 2-para-chlorophényl 2-isopropyl acétiques, par les esters d'alléthrolones, d'alcool 3,4,5,6-tétrahydrophtalimidométhylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahaloéthyl) cyclopropane-1-carboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

L'invention a également pour objet un procédé de préparation des composés de formule (I) caractérisé en ce que l'on soumet un acide de formule (II) : 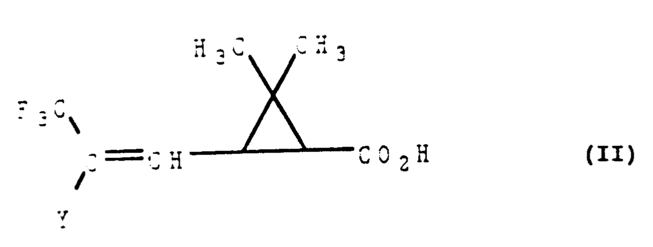
dans laquelle Y conserve sa signification précédente ou un dérivé fonctionnel de cet acide, à l'action d'un alcool de formule (III) : 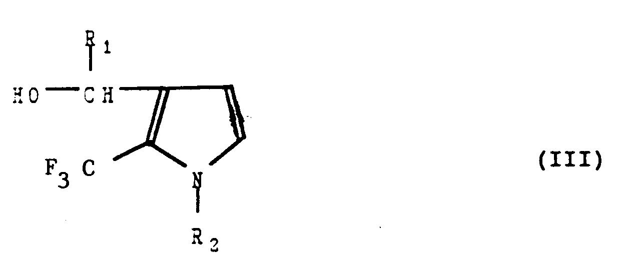
dans laquelle R₁ et R₂ conservent leur signification précédente pour obtenir le composé de formule (I) correspondant.

Les composés de formule (II) sont des produits connus (cf par exemple EP 0335801, 0133406, 19787, 10859, 61114 ou le brevet français 2185612).

Les alcools de formule (III) sont également des produits connus (par exemple dans le brevet européen 0176386).

Les exemples suivants illustrent l'invention sans toutefois la limiter.

### EXEMPLE 1 : [1R-[1alpha,3alpha(Z)]]-3-(2-chloro 3,3,3-trifluoro 1-propényl) 2,2-diméthyl cyclopropanecarborylate de [1-(2-propynyl) 2-(trifluorométhyl) 1H-pyrrol-3-yl] méthyle,

On refroidit à 0°C une solution renfermant 450 mg de 1-(2-propynyl) 2-(trifluorométhyl) 1H-pyrrol-3-méthanol, 410 mg d'acide [1R-[1alpha,3alpha(Z)]]-3-(2-chloro 3,3,3-trifluoro 1-propényl) 2,2-diméthyl cyclopropanecarboxylique et 8 cm³ de chlorure de méthylène anhydre. On ajoute à 0°, à la solution ainsi préparée, une solution renfermant 345 mg de dicyclohexylcarbodiimide. 20 mg de diméthylaminopyridine et 10 cm³ de chlorure de méthylène. On laisse le mélange réactionnel revenir à la température ambiante et le maintient sous agitation pendant 2 heures. On filtre, concentre le filtrat et le reprend dans l'éther isopropylique. On élimine l'insoluble par filtration. On concentre le filtrat ainsi obtenu. On le chromatographie sur silice en éluant avec le mélange hexaneacétate d'éthyle (9-1). On obtient 618 mg de produit, rdt 85 %.
[alpha]_{D} = + 15°5 ± 2° (c = 0,5 %, CHCl₃):

### Exemple 2 : Préparation d'un concentré soluble

On effectue un mélange homogène de :

| | |
|---|---|
| Produit de l'exemple 1 | 0,25 g |
| Butoxyde pipéronyle | 1,00 g |
| Tween 80 | 0,25 g |
| Topanol A | 0,1 g |
| Eau | 98,4 g |

### Exemple 3 : Préparation d'un concentré émulsifiable

On effectue un mélange homogène de :

| | |
|---|---|
| Produit de l'exemple 1 | 1,5 g |
| Tween 80 | 20,00 g |
| Topanol A | 0,1 g |
| Xylène | 78,4 g |

### Exemple 4 : Préparation d'une composition fumigène

On mélange d'une façon homogène :

| | |
|---|---|
| Produit de l'exemple 1 | 0,25 g |
| Poudre de tabu | 25,00 g |
| Poudre de feuille de cèdre | 40,00 g |
| Poudre de bois de pin | 33,75 g |
| Vert brillant | 0,5 g |
| p-Nitrophénol | 0,5 g |

### ETUDE BIOLOGIQUE

### A. Etude de l'effet d'abattage sur mouche domestique

Les insectes tests sont des mouches domestiques femelles âgées de 4 jours. On opère par pulvérisation directe à la concentration de 0,10 g/l ou 1 g/l en chambre de Kearns et March en utilisant comme solvant un mélange d'acétone (5 %) et d'Isopar L (solvant pétrolier) (quantité de solvant utilisée 2 cm³ en une seconde). On utilise 50 insectes par traitement. On effectue les contrôles toutes les minutes jusqu'à 10 minutes, puis à 15 minutes et l'on détermine le KT₅₀ par les méthodes habituelles.

Les résultats expérimentaux obtenus sont résumés dans le tableau suivant :

| Composés | Concentration (g/l) | KT₅₀ en mn |
|---|---|---|
| Exemple 1 | 0,1 | 10,4 |

### B. Etude de l'effet létal des composés de l'invention sur divers insectes

### a) Etude de l'effet létal sur mouche domestique

Les insectes tests sont des mouches domestiques femelles âgées de 4 à 5 jours. On opère par application topique de 1 microlitre de solution acétonique sur le thorax dorsal des insectes à l'aide du micro manipulateur d'Arnold. On utilise 30 individus par traitement. On effectue le contrôle de mortalité vingt-quatre heures après traitement.
Les résultats obtenus exprimés en DL₅₀ ou dose (en nanogrammes) par individu nécessaire pour tuer 50 % des insectes, sont les suivants :

| Composés | DL₅₀ en ng/insecte |
|---|---|
| Exemple 1 | 8,3 |

### b) Etude de l'effet létal sur larves de Spodoptera Littoralis

Les essais sont effectués par application topique d'une solution acétonique à l'aide du micro manipulateur d'Arnold sur le thorax dorsal des larves. On utilise 15 larves par dose de produit à tester. Les larves utilisées sont des larves du quatrième stade larvaire, c'est-à-dire âgées d'environ 10 jours lorsqu'elles sont élevées à 24°C et 65 % d'humidité relative. Après traitement les individus sont placés sur milieu nutritif artificiel (milieu de Poitout).

On effectue le contrôle des mortalités 48 heures après traitement.
Les résultats expérimentaux obtenus sont résumés dans le tableau suivant :

| Composés | DL₅₀ en ng/insecte |
|---|---|
| Exemple 1 | 11,2 |

### c) Etude de l'activité sur Diabrotica

Les insectes tests sont des larves de dernier stade de Diabrotica undécimpunctata.
On traite une rondelle de papier filtre de 9 cm de diamètre, déposée au fond d'une boite de Petri, à l'aide de 1 cm³ de solution acétonique. Après séchage on dépose 15 larves par dose et on effectue le contrôle de mortalité 24 h après le traitement.
On détermine la dose létale 100 (DL₁₀₀) exprimée en mg/litre. Les résultats obtenus sont les suivants :
Produit de l'exemple 1 : 0,6

### C) ETUDE DE L'ACTIVITE SUR APHIS CRACCIVORA

On pulvérise 2 cm³ de solution hydro-acétonique (50/50) sur des feuilles de fève (vicia fabae) jusqu'à ruissellement. Après séchage, on dépose 15 femelles aptères d'Aphis cracivora. L'ensemble est maintenu sur du papier filtre humide à l'intérieur d'une boîte Pétri. 24 heures après le début du contact, on procède à la détermination du nombre d'insectes morts.

| Composés | CL₅₀ en mg/l |
|---|---|
| Exemple 1 | 0,15 |

## Revendications (Revendications pour l'(les) Etat(s) contractant(s) suivant(s): AT, BE, CH, DE, DK, FR, GB, GR, IT, LI, LU, NL, SE)

1. Sous toutes leurs formes stéréoisomères possibles, ainsi que leurs mélanges, les composés de formule (I) : 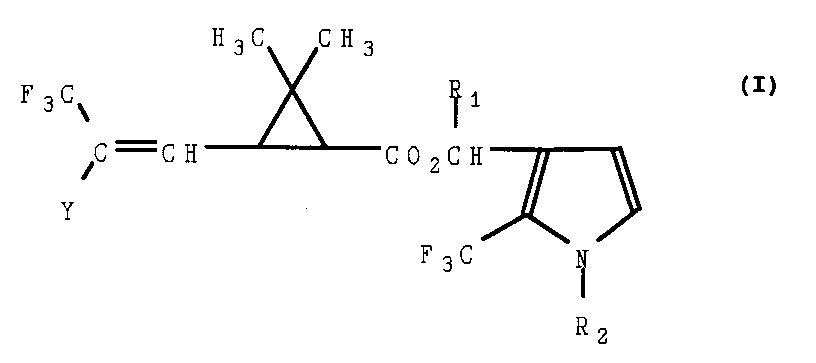 dans lesquels :
Y représente un atome d'hydrogène, un atome d'halogène, un radical cyano, un radical alkyle éventuellement substitué par un ou plusieurs atomes d'halogène, renfermant jusqu'à 8 atomes de carbone, un radical phényle éventuellement substitué par un ou plusieurs atomes d'halogène et/ou un ou plusieurs radicaux alkyle renfermant jusqu'à 8 atomes de carbone et/ou plusieurs radicaux alkoxy renfermant jusqu'à 8 atomes de carbone,
- R₁ représente un atome d'hydrogène, un radical méthyle, un radical cyano ou un radical éthynyle,
- R₂ représente un radical : 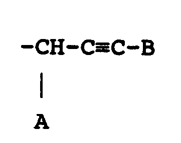 dans lequel A et B identiques ou différents l'un de l'autre, représentent un atome d'hydrogène, un atome d'halogène, un radical alkyle, renfermant jusqu'à 8 atomes de carbone ou un radical aryle renfermant jusqu'à 14 atomes de carbone.

2. Les composés de formule (I) définis à la revendication 1, dans lesquels Y représente un atome de chlore.

3. Les composés de formule (I) définis à l'une quelconque des revendications 1 et 2 dans lesquels R₁ représente un atome d'hydrogène.

4. Les composés de formule (I) définis à l'une quelconque des revendications 1 à 3, dans lesquels R₂ représente un radical propargyle.

5. Les composés de formule (I) définis à l'une quelconque des revendications 1 à 4, dans lesquels la copule cyclopropanique est de structure (1R,cis).

6. Le composé répondant à la formule (I) de la revendication 1, dont le nom suit :
- le [1R-[1alpha,3alpha(Z)]]-3-(2-chloro 3,3,3-trifluoro 1-propényl) 2,2-diméthyl cyclopropanecarboxylate de [1-(2-propynyl) 2-(trifluorométhyl) 1H-pyrrol-3-yl] méthyle.

7. Les compositions pesticides renfermant comme principe actif au moins un composé de formule (I) défini à l'une quelconque des revendications 1 à 4.

8. Les compositions pesticides renfermant comme principe actif le produit défini à la revendication 6.

9. Les compositions pesticides définies à la revendication 7 ou 8 caractérisées en ce qu'elles sont destinées à lutter contre DIABROTICA et les autres insectes parasites du sol.

10. Procédé de préparation des composés de formule (I) définis à l'une quelconque des revendications 1 à 5, caractérisé en ce que l'on soumet un acide de formule (II) : 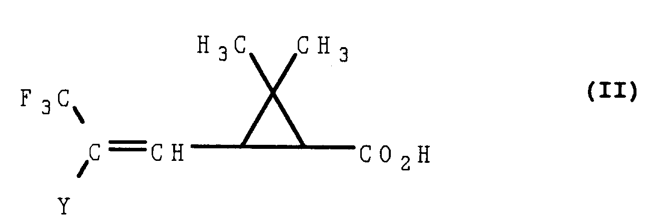 dans laquelle Y conserve sa signification précédente ou un dérivé fonctionnel de cet acide, à l'action d'un alcool de formule (III) : 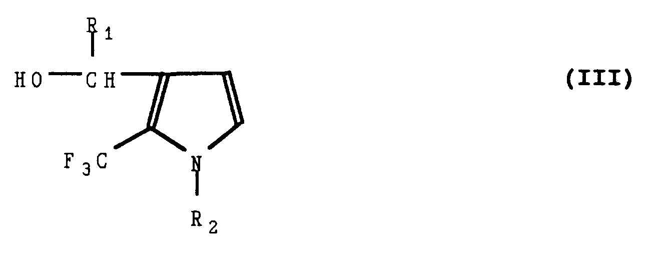 dans laquelle R₁ et R₂ conservent leur signification précédente pour obtenir le composé de formule (I) correspondant.

## Revendications (Revendications pour l'(les) Etat(s) contractant(s) suivant(s): ES)

1. Procédé de préparation des composés de formule (I) : 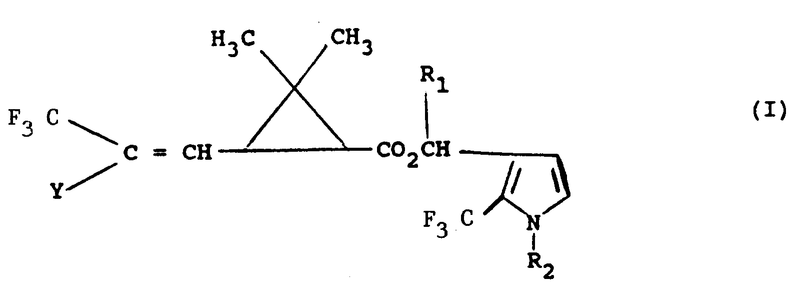 dans lesquels :
- Y représente un atome d'hydrogène, un atome d'halogène, un radical cyano, un radical alkyle éventuellement substitué par un ou plusieurs atomes d'halogène, renfermant jusqu'à 8 atomes de carbone, un radical phényle éventuellement substitué par un ou plusieurs atomes d'halogène et/ou un ou plusieurs radicaux alkyle renfermant jusqu'à 8 atomes de carbone et/ou plusieurs radicaux alkoxy renfermant jusqu'à 8 atomes de carbone,
- R₁ représente un atome d'hydrogène, un radical méthyle, un radical cyano ou un radical éthynyle,
- R₂ représente un radical : 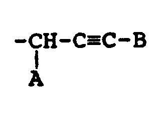 dans lequel A et B identiques ou différents l'un de l'autre, représentent un atome d'hydrogène, un atome d'halogène, un radical alkyle, renfermant jusqu'à 8 atomes de carbone ou un radical aryle renfermant jusqu'à 14 atomes de carbone, sous toutes leurs formes stéréoisomères possibles, ainsi que leurs mélanges, caractérisé en ce que l'on soumet un acide de formule (II) : 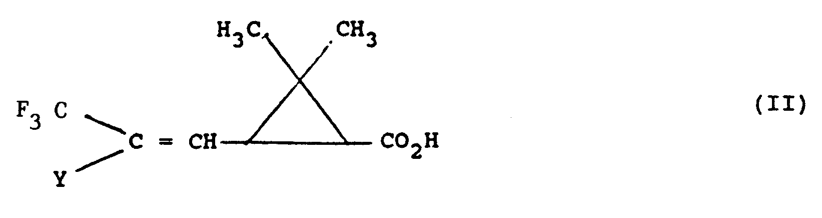 dans laquelle Y conserve sa signification précédente ou un dérivé fonctionnel de cet acide, à l'action d'un alcool de formule (III) : 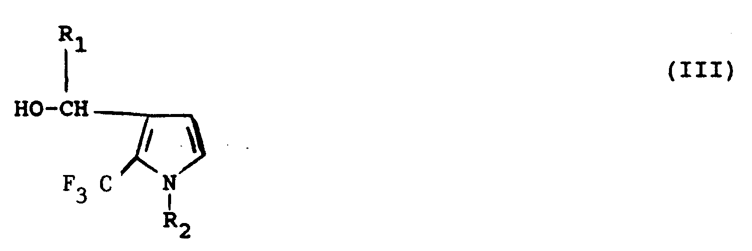 dans laquelle R₁ et R₂ conservent leur signification précédente pour obtenir le composé de formule (I) correspondant.

2. Procédé selon la revendication 1, caractérisé en ce que l'on utilise au départ un produit de formule (II) dans laquelle Y représente un atome de chlore.

3. Procédé selon la revendication 1, caractérisé en ce que l'on utilise au départ un produit de formule (III) dans laquelle R₁ représente un atome d'hydrogène.

4. Procédé selon la revendication 1, caractérisé en ce que l'on utilise au départ un produit de formule (III) dans laquelle R₂ représente un radical propargyle.

5. Procédé selon la revendication 1, caractérisé en ce que l'on utilise au départ un produit de formule (II) dans laquelle la copule cyclopropanique est de structure (1R,cis).

## Claims (Claims for the following Contracting State(s): AT, BE, CH, DE, DK, FR, GB, GR, IT, LI, LU, NL, SE)

1. In all their possible stereoisomer forms, as well as their mixtures, the compounds of formula (I): 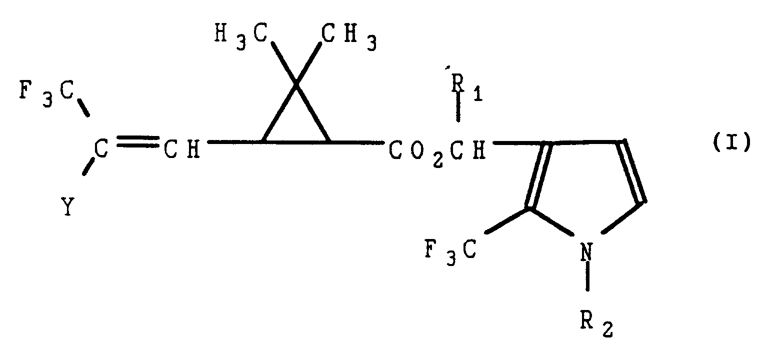 in which:
Y represents a hydrogen atom, a halogen atom, a cyano radical, an alkyl radical optionally substituted by one or more halogen atoms, containing up to 8 carbon atoms, a phenyl radical optionally substituted by one or more halogen atoms and/or one or more alkyl radicals containing up to 8 carbon atoms and/or several alkoxy radicals containing up to 8 carbon atoms,
- R₁ represents a hydrogen atom, a methyl radical, a cyano radical or an ethynyl radical,
- R₂ represents a radical: 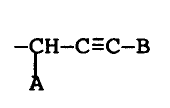 in which A and B, identical to or different from each other, represent a hydrogen atom, a halogen atom, an alkyl radical containing up to 8 carbon atoms or an aryl radical containing up to 14 carbon atoms.

2. The compounds of formula (I) defined in claim 1, in which Y represents a chlorine atom.

3. The compounds of formula (I) defined in any one of claims 1 and 2 in which R₁ represents a hydrogen atom.

4. The compounds of formula (I) defined in any one of claims 1 to 3, in which R₂ represents a propargyl radical.

5. The compounds of formula (I) defined in any one of claims 1 to 4, in which the cyclopropane copula is of (1R,cis) structure.

6. The compound corresponding to formula (I) of claim 1, the name of which follows:
- [1-(2-propynyl) 2-(trifluoromethyl) 1H-pyrrol-3-yl] methyl [1R-[1alpha,3alpha(Z)]]-3-(2-chloro 3,3,3-trifluoro 1-propenyl) 2,2-dimethyl cyclopropanecarboxylate.

7. The pesticide compositions containing as active ingredient at least one compound of formula (I) defined in any one of claims 1 to 4.

8. The pesticide compositions containing as active ingredient the product defined in claim 6.

9. The pesticide compositions defined in claim 7 or 8 characterized in that they are intended for combating DIABROTICA and other parasitic insects of the soil.

10. Preparation process for the compounds of formula (I) defined in any one of claims 1 to 5, characterized in that an acid of formula (II): 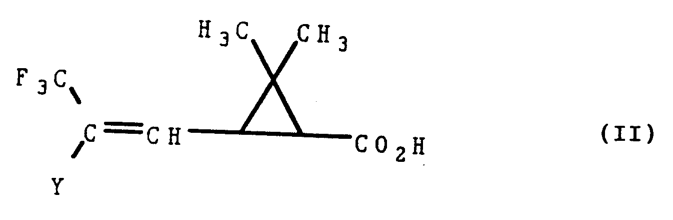 in which Y retains its previous meaning or a functional derivative of this acid, is subjected to the action of an alcohol of formula (III): 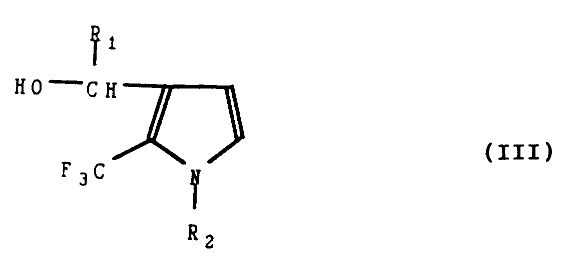 in which R₁ and R₂ retain their previous meaning, in order to obtain the corresponding compound of formula (I).

## Claims (Claims for the following Contracting State(s): ES)

1. Preparation process for compounds of formula (I): 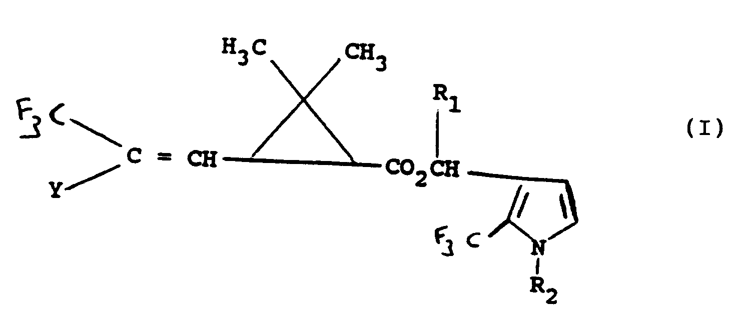 in which:
Y represents a hydrogen atom, a halogen atom, a cyano radical, an alkyl radical optionally substituted by one or more halogen atoms, containing up to 8 carbon atoms, a phenyl radical optionally substituted by one or more halogen atoms and/or one or more alkyl radicals containing up to 8 carbon atoms and/or several alkoxy radicals containing up to 8 carbon atoms,
- R₁ represents a hydrogen atom, a methyl radical, a cyano radical or an ethynyl radical,
- R₂ represents a radical: 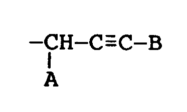 in which A and B, identical to or different from each other, represent a hydrogen atom, a halogen atom, an alkyl radical containing up to 8 carbon atoms or an aryl radical containing up to 14 carbon atoms, in all their possible stereoisomer forms, as well as their mixtures, characterized in that an acid of formula (II): 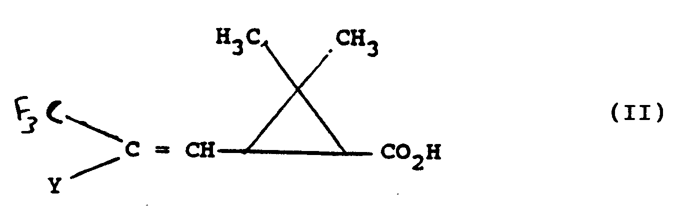 in which Y retains its previous meaning, or a functional derivative of this acid, is subjected to the action of an alcohol of formula (III): 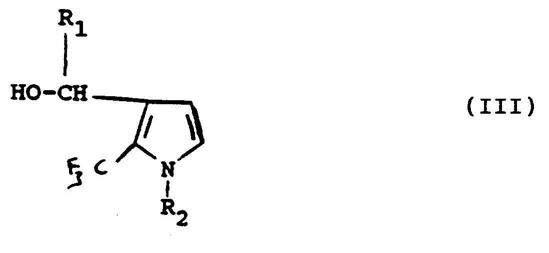 in which R₁ and R₂ retain their previous meaning, in order to obtain the corresponding compound of formula (I).

2. Process according to 1, characterized in that a product of formula (II) is used at the start in which Y represents a chlorine atom.

3. Process according to claim 1, characterized in that a product of formula (III) is used at the start in which R₁ represents a hydrogen atom.

4. Process according to claim 1, characterized in that a product of formula (III) is used at the start in which R₂ represents a propargyl radical.

5. Process according to claim 1, characterized in that a product of formula (II) is used at the start in which the cyclopropane copula is of (1R,cis) structure.

## Patentansprüche (Patentansprüche für folgende(n) Vertragsstaat(en): AT, BE, CH, DE, DK, FR, GB, GR, IT, LI, LU, NL, SE)

1. Verbindungen der Formel (I) in allen ihren möglichen stereoisomeren Formen sowie deren Mischungen: 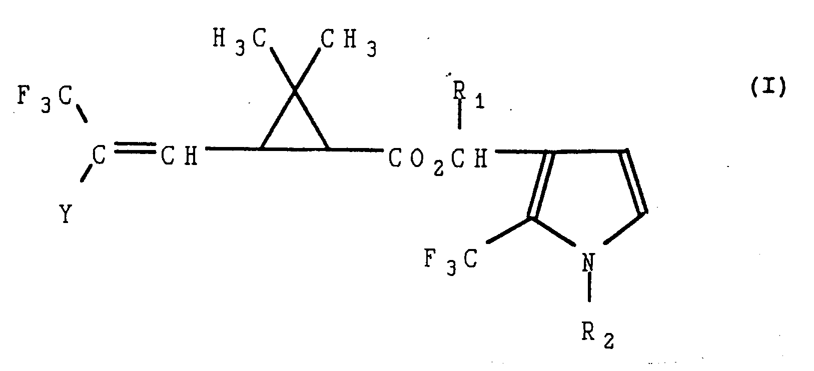 in denen:
Y ein Wasserstoffatom, ein Halogenatom, einen Cyanorest, einen gegebenenfalls durch ein oder mehrere Halogenatome substituierten Alkylrest, der bis zu 8 Kohlenstoffatome umfaßt, einen gegebenenfalls durch ein oder mehrere Halogenatome und/oder einen oder mehrere bis zu 8 Kohlenstoffatome umfassende Alkylreste und/oder mehrere bis zu 8 Kohlenstoffatome umfassende Alkoxyreste substituierten Phenylrest darstellt,
- R₁ ein Wasserstoffatom, einen Methylrest, einen Cyanorest oder einen Ethinylrest darstellt,
- R₂ einen Rest: 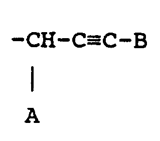 darstellt, in dem A und B, identisch oder verschieden voneinander, ein Wasserstoffatom, ein Halogenatom, einen bis zu 8 Kohlenstoffatome umfassenden Alkylrest oder einen bis zu 14 Kohlenstoffatome umfassenden Arylrest darstellen.

2. Verbindungen der Formel (I) nach Anspruch 1, in denen Y ein Chloratom darstellt.

3. Verbindungen der Formel (I) nach irgendeinem der Ansprüche 1 und 2, in denen R₁ ein Wasserstoffatom darstellt.

4. Verbindungen der Formel (I) nach irgendeinem der Ansprüche 1 bis 3, in denen R₂ einen Propargylrest darstellt.

5. Verbindungen der Formel (I) nach irgendeinem der Ansprüche 1 bis 4, in denen das Cyclopropanglied die Struktur (1R, cis) aufweist.

6. Verbindung gemäß der Formel (I) des Anspruchs 1, deren Name folgt:
- [1-(2-Propinyl)-2-(trifluormethyl)-1H-pyrrol-3-yl]methyl-[1R-[1alpha,3alpha(Z)]]-3-(2-chlor-3,3,3-trifluor-1-propenyl)-2,2-dimethylcyclopropancarboxylat.

7. Pestizide Zusammensetzungen, die als Wirkstoff mindestens eine Verbindung der Formel (I), definiert in irgendeinem der Ansprüche 1 bis 4, umfassen.

8. Pestizide Zusammensetzungen, die als Wirkstoff das in Anspruch 6 definierte Produkt umfassen.

9. Pestizide Zusammensetzungen nach Anspruch 7 oder 8, dadurch gekennzeichnet, daß sie dazu bestimmt sind, DIABROTICA und die anderen parasitischen Insekten des Bodens zu bekämpfen.

10. Verfahren zur Herstellung der Verbindungen der Formel (I) nach irgendeinem der Ansprüche 1 bis 5, dadurch gekennzeichnet, daß man eine Säure der Formel (II): 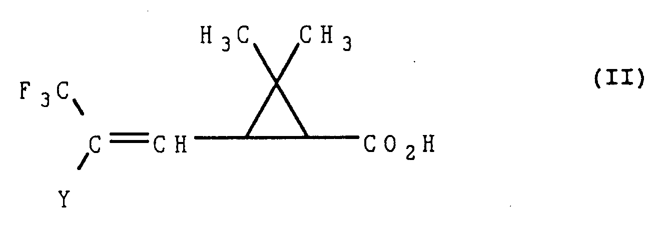 in der Y die vorstehende Bedeutung aufweist, oder ein funktionelles Derivat dieser Säure der Einwirkung eines Alkohols der Formel (III): 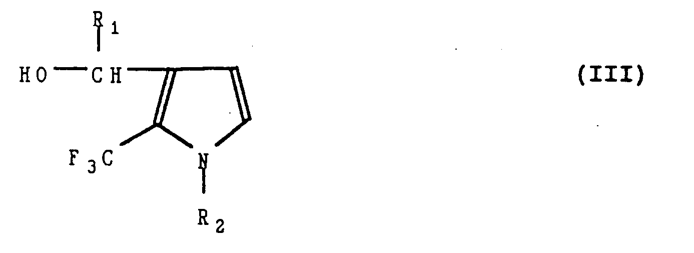 in der R₁ und R₂ ihre vorstehende Bedeutung beibehalten, unterzieht, um die entsprechende Verbindung der Formel (I) zu erhalten.

## Patentansprüche (Patentansprüche für folgende(n) Vertragsstaat(en): ES)

1. Verfahren zur Herstellung von Verbindungen der Formel (I): 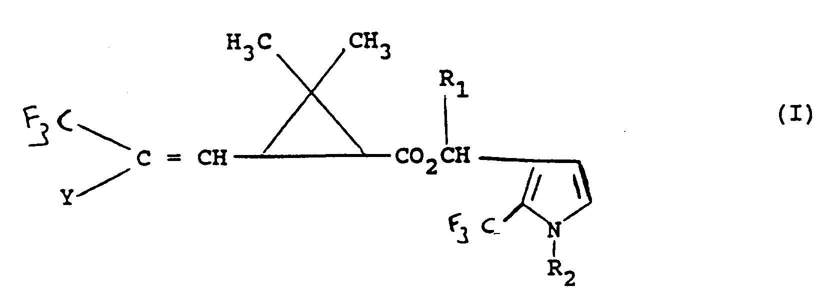 in denen:
Y ein Wasserstoffatom, ein Halogenatom, einen Cyanorest, einen gegebenenfalls durch ein oder mehrere Halogenatome substituierten Alkylrest, der bis zu 8 Kohlenstoffatome umfaßt, einen gegebenenfalls durch ein oder mehrere Halogenatome und/oder einen oder mehrere bis zu 8 Kohlenstoffatome umfassende Alkylreste und/oder mehrere bis zu 8 Kohlenstoffatome umfassende Alkoxyreste substituierten Phenylrest darstellt,
- R₁ ein Wasserstoffatom, einen Methylrest, einen Cyanorest oder einen Ethinylrest darstellt,
- R₂ einen Rest: 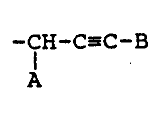 darstellt, in dem A und B, identisch oder verschieden voneinander, ein Wasserstoffatom, ein Halogenatom, einen bis zu 8 Kohlenstoffe umfassenden Alkylrest oder einen bis zu 14 Kohlenstoffatome umfassenden Arylrest darstellen,
in allen ihren möglichen stereoisomeren Formen sowie deren Mischungen,
dadurch gekennzeichnet, daß man eine Säure der Formel (II): 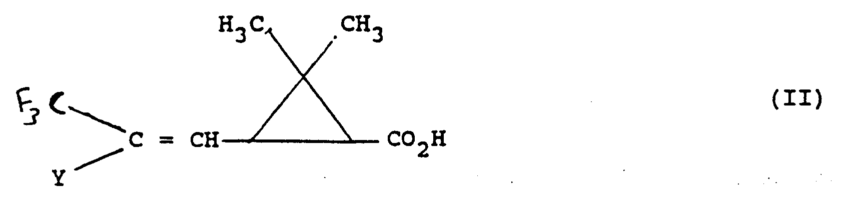 in der Y die vorstehende Bedeutung beibehält, oder ein funktionelles Derivat dieser Säure der Einwirkung eines Alkohols der Formel (III): 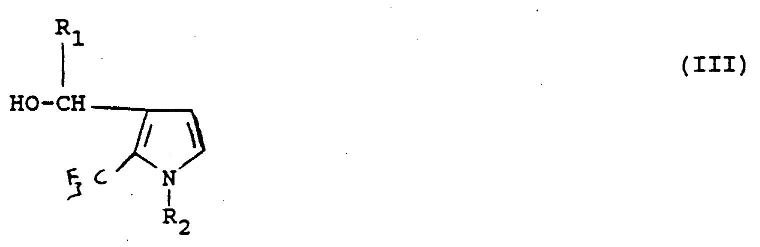 in der R₁ und R₂ ihre vorstehende Bedeutung beibehalten, unterzieht, um die entsprechende Verbindung der Formel (I) zu erhalten.

2. Verfahren nach Anspruch 1, dadurch gekennzeichnet, daß man zu Beginn ein Produkt der Formel (II), in der Y ein Chloratom darstellt, verwendet.

3. Verfahren nach Anspruch 1, dadurch gekennzeichnet, daß man zu Beginn ein Produkt der Formel (III), in der R₁ ein Wasserstoffatom darstellt, verwendet.

4. Verfahren nach Anspruch 1, dadurch gekennzeichnet, daß man zu Beginn ein Produkt der Formel (III), in der R₂ einen Propargylrest darstellt, verwendet.

5. Verfahren nach Anspruch 1, dadurch gekennzeichnet, daß man zu Beginn ein Produkt der Formel (II), in der das Cyclopropanglied die Struktur (1R, cis) aufweist, verwendet.
